Europäisches Patentamt

European Patent Office

Office européen des brevets

(19)

(11) Veröffentlichungsnummer: **0 167 122
B1**

# (12) EUROPÄISCHE PATENTSCHRIFT

(45) Veröffentlichungstag der Patentschrift: **04.04.90**

(21) Anmeldenummer: **85108010.1**

(22) Anmeldetag: **28.06.85**

(51) Int. Cl.⁵: **A 61 N 1/39,** A 61 B 5/04,
H 05 K 5/00

(54) **Tragbares elektromedizinisches Gerät.**

(30) Priorität: **06.07.84 DE 3424906**

(43) Veröffentlichungstag der Anmeldung:
**08.01.86 Patentblatt 86/02**

(45) Bekanntmachung des Hinweises auf die
Patenterteilung:
**04.04.90 Patentblatt 90/14**

(84) Benannte Vertragsstaaten:
**FR GB NL**

(56) Entgegenhaltungen:
**US-A-4 097 113**

(73) Patentinhaber: **O.D.A.M. - OFFICE DE
DISTRIBUTION D'APPAREILS MEDICAUX
34, Rue de l'Industrie
F-67160 Wissembourg (FR)**

(72) Erfinder: **Reithler, Jean-Claude
24, Rue de la Laine
F-67160 Wissembourg (FR)**

(74) Vertreter: **Patentanwälte Kohler - Schwindling -
Späth
Hohentwielstrasse 41
D-7000 Stuttgart 1 (DE)**

Courier Press, Leamington Spa, England.

EP 0 167 122 B1

**Beschreibung**

Die Erfindung bezieht sich auf ein tragbares elektromedizinisches Gerät, bestehend aus mehreren Geräteteilen, beispielsweise einer Monitor-Schreibereinheit und einem Defibrillator, wobei die Geräteteile mindestens näherungsweise quaderförmig mit einer Vorderwand, einer Rückwand und zwei Seitenwänden ausgebildet und im Bereich der Wände mittels Steckverbindungen mechanisch und elektrisch verbindbar sind.

Ein derartiges Gerät ist durch das Manual Nr. 09-10427-07 "LIFEPAK 5" der Firma Physio Control bekannt geworden.

Das bekannte Gerät besteht aus mehreren quaderförmigen Geräteteilen, die jeweils an der Vorderseite mit einem starren, über die gesamte Gerätebreite gehenden Griff versehen sind. Die Geräteteile lassen sich seitlich miteinander über eine Steckverbindung koppeln, die als Schieber mit Rastung ausgebildet ist. Die Seitenwände der Geräteteile sind dabei mit freiliegenden Kontakten versehen, die beim Verbinden der Geräteteile in Anlage zueinander geraten, jedoch lediglich Signalspannungen, nicht jedoch eine Versorgungsspannung übertragen.

Schließlich bestehen die Geräteteile des bekannten Gerätes aus -- abgesehen von den Verbindungselementen - unterschiedlichen Gehäuseteilen.

Tragbare elektromedizinische Geräte werden im allgemeinen für den mobilen Einsatz, insbesondere für den Notfalleinsatz, verwendet. Bei diesen Anwendungsfällen kommt es jedoch nicht nur auf ein geringes Gewicht der Geräte, sondern ebenso auf möglichst kompakte Abmessungen an, weil derartige Geräte häufig an Bord von Krankenwagen, Notarztwagen, Rettungshubschraubern u. dgl. zum Einsatz kommen oder in Kliniken in fahrbare Notfallwagen, sogenannte Herzalarmwagen, eingebaut werden.

Geräte der eingangs genannten Art, bei denen Geräteteile miteinander mechanisch und elektrisch verbindbar sind, haben in diesem Zusammenhang den Vorteil, daß man das Gesamtsystem "mit einem Griff" zur Verfügung hat und gleichzeitig alle erforderlichen Signalverbindungen hergestellt sind, die man beispielsweise zwischen einem kardiologischen Monitor und einem Defibrillator benötigt, um den Defibrillator synchronisiert mit der Herztätigkeit des Patienten, die vom Monitor erfaßt wird, auslösen zu können. Außerdem kann man mit einer solchen kompletten, aus mehreren Geräteteilen bestehenden Einheit die Defibrillatorelektroden als EKG-Elektroden verwenden und entweder das EKG-Signal auf einem Monitorbildschirm darstellen oder beispielsweise die Pulsfrequenz als Zahlenwert anzeigen. Auch kann man bei entsprechender Kombination der Einheit das EKG-Signal auf einem Schreiber dokumentieren.

Andererseits haben diese Geräte auch den Vorteil, daß die Geräteteile auch einzeln und separat genutzt werden können, beispielsweise kann während eines Notarzteinsatzes bei einem Verkehrsunfall das Gerät in seine Geräteteile getrennt werden, und die einzelnen Geräteteile können für verschiedene Unfallopfer benutzt werden.

Das eingangs genannte bekannte Gerät genügt jedoch noch nicht allen Anforderungen, die man an derartige Geräte stellen könnte. Zum einen widerspricht es der Forderung nach minimalen Außenabmessungen, daß bei dem bekannten Gerät im verbundenen Zustand der Geräteteile beide Griffe der Geräteteile vorstehen und platz beanspruchen, obwohl ein einziger Griff ausreichen würde. Weiterhin haben die freiliegenden Kontakte für die Signalspannungen den Nachteil, daß elektrische Störungen von außen in die Geräte eindringen und beispielsweise Fehlsynchronisationen oder Signalverfälschungen hervorrufen können. Weiterhin hat das bekannte Gerät den Nachteil, daß mangels der Möglichkeit einer Versorgungsspannungsübertragung von einem Geräteteil auf das andere jedes Geräteteil mit einer voll funktionierenden Stromversorgungseinheit ausgestattet sein muß und beispielsweise bei erschöpfter Batterie des einen Geräteteils das andere Geräteteil nicht zur Stromversorgung und/oder zum Nachladen der Batterie herangezogen werden kann. Schließlich ist bei dem bekannten Gerät noch von Nachteil, daß durch die Verschiedenheit der Gehäuseteile sowohl die Herstellung wie auch die Lagerhaltung beim Kundendienst verkompliziert und verteuert wird.

Der Erfindung liegt demgegenüber die Aufgabe zugrunde, ein Gerät der eingangs genannten Art dahingehend weiterzubilden, daß bei minimalem Platzbedarf des aus Geräteteilen zusammengesetzten Gerätes ein Höchstmaß an elektrischer Sicherheit gewährleistet ist sowohl hinsichtlich möglicher Einstreuungen von Störspannungen wie auch hinsichtlich der Stromversorgung und daß schließlich Herstellung und Wartung des Gerätes vereinfacht und damit verbilligt werden.

Diese Aufgabe wird erfindungsgemäß dadurch gelöst, daß die Seitenwände im an die Vorderwand angrenzenden Bereich mit seitlichen Laschen versehen sind, die über die Vorderwand vorstehen und dort an ihren freien Enden über einen durchgehenden Griff verbunden sind, daß die Vorderwand und/oder die Rückwand mit einer parallel zum Griff verlaufenden Kehle versehen ist und daß der Griff in den Laschen verschiebbar angeordnet ist, derart, daß beim Verbinden zweier Geräteteile die freien Enden der Laschen eines Geräteteiles die Seitenwände des anderen Geräteteiles umfassen und der Griff in den Bereich der Kehle gelangt.

Das erfindungsgemäße Gerät hat damit den wesentlichen Vorteil, daß das aus Geräteteilen zusammengesetzte Gesamtgerät nur noch einen einzigen Griff an der Vorderseite aufweist, mit dem das Gerät aus einer Halterung oder einem Regal herausgenommen und getragen werden kann. Die Griffe der anderen Geräteteile befinden sich unterdessen im in die Kehlen eingeschobenen Zustand und beanspruchen keinen zusätzlichen Platz. Trennt man die Geräteteile wieder voneinander, besitzt jedes Geräteteil wieder seinen eigenen Griff und kann für sich allein gehandhabt werden.

Gemäß einer Weiterbildung der Erfindung wird der Griff beim Verbinden zweier Geräteteile gegen die Kraft einer Feder geführt.

Diese Maßnahme hat den Vorteil, daß beim Lösen der Verbindung zweier Geräteteile der eingefahrene Griff des einen Geräteteils von selbst durch Federkraft nach außen springt und damit sofort umfaßt werden kann.

Zweckmäßigerweise läuft der Griff beim erfindungsgemäßen Gerät mit an seinen Enden angeordneten Stiften in Führungsnuten der Laschen.

Bei einer weiteren Ausführungsform der Erfindung sind die Laschen mit mechanischen und/oder elektrischen Verbindungsmitteln versehen, die mit zugeordneten mechanischen und/oder elektrischen Verbindungsmitteln im an die Rückwand angrenzenden Bereich der Seitenwände eines anderen Geräteteils zusammenarbeiten.

Diese Maßnahme hat den Vorteil, daß ein oder mehrere der elektrischen und/oder mechanischen Verbindungsmittel an der Innenseite der Laschen angeordnet sind und somit von außen praktisch nicht zugänglich sind. Ein versehentliches Kurzschließen der Verbindungsmittel durch seitliche Berührung mit einem elektrisch leitenden Gegenstand ist damit ausgeschlossen.

Bei einem bevorzugten Ausführungsbeispiel sind die mechanischen Verbindungsmittel als mindestens eine elastische Rastnase in einem Geräteteil ausgebildet. die mit einer Aussparung in einem anderen Geräteteil zusammenarbeitet.

Diese Anordnung gewährleistet einen besonders sicheren mechanischen Sitz bei gleichzeitig unkompliziertem und damit wenig störungsanfälligem Aufbau.

Die letztgenannte Ausführungsform wird bevorzugt dadurch weitergebildet. daß die Rastnasen in den Laschen angeordnet sind, wobei die Rastnasen in einer Führung schräg zur Verbindungsrichtung der Geräteteile mittels eines Knopfes gegen die Kraft einer Feder außer Eingriff mit der Aussparung bewegbar sind.

Diese Maßnahme hat zum einen den Vorteil, daß die mechanische Verbindung besonders leicht herzustellen und sicher ist, weil die Rastnasen in dem Bereich wirken, in dem die Laschen das jeweils andere Geräteteil seitlich umfassen.

Außerdem haben die genannten Merkmale den Vorteil, daß die mechanische Rastverbindung durch einfaches Betätigen des Knopfes besonders leicht gelöst werden kann, eine Eigenschaft, die gerade bei Notfallgeräten von besonderer Bedeutung ist.

Bei einer weiteren bevorzugten Ausgestaltung der Erfindung sind die elektrischen Verbindungsmittel als Kontaktfedern in einem Geräteteil ausgebildet, die mit Kontaktnieten oder Kontaktbahnen in einem anderen Geräteteil zusammenarbeiten.

Diese Maßnahme hat den Vorteil, daß durch geeignete Einstellung des Kontaktdruckes der Federn die Kontaktgabel beim Einschieben und im verbundenen Zustand der Geräteteile sicher gewährleistet werden kann. Außerdem haben die Kontaktnieten und Kontaktbahnen den Vorteil, daß sie im wesentlichen stetig aus der Oberfläche des Gerätes hervortreten können, so daß Verschmutzungen und andere Ablagerungen nicht zu befürchten sind.

Bei einer besonders bevorzugten Ausgestaltung der Erfindung ist im Bereich der Lasche ein beim Verbinden zweier Geräteteile betätigter Schalter angeordnet, dessen Schaltkontakte in Reihe mit den elektrischen Verbindungsmitteln liegen.

Diese Maßnahme hat den wesentlichen Vorteil, daß z.B. diejenigen elektrischen Verbindungsmittel, an denen Signaloder Versorgungsspannungen anstehen, bei nicht verbundenen Geräteteilen stromlos gemacht werden können und diejenigen elektrischen Verbindungsmittel, die als Eingangsklemmen dienen, von den übrigen elektrischen Baueinheiten des Geräteteiles abgetrennt werden. Es können daher weder Signal- noch Versorgungsausgänge kurzgeschlossen noch Signaleingänge mit Störspannungen beaufschlagt werden, weil die jeweiligen elektrischen Verbindungsmittel erst dann zugeschaltet werden, wenn die Geräteteile miteinander verbunden sind.

Bevorzugt ist der Schalter dabei als Mikroschalter ausgebildet, dessen Schaltfühler mit der Kontaktfeder ausgelenkt wird.

Dies hat den Vorteil, daß besonders wenig Bauteile erforderlich sind, weil die ohnehin auftretende Auslenkung der Kontaktfeder gleichzeitig zum Betätigen des Mikroschalters herangezogen wird.

Man kann jedoch in einer anderen Ausgestaltung der Erfindung den Schalter auch als Mikroschalter ausbilden, dessen Schaltfühler beim Zusammenlaufen der Lasche eines Geräteteiles mit einer auf einem anderen Geräteteil angeordneten Haube, die bei verbundenen Geräteteilen an die Lasche angrenzt, ausgelenkt wird.

Diese Ausführungsform eignet sich besonders für diejenigen Fälle, bei denen große Mikroschalter verwendet werden müssen, deren Betätigungskraft und/oder Betätigungsweg größer ist als dies beim vorgenannten Ausführungsbeispiel der Fall war.

Bei einer anderen Ausgestaltung der Erfindung dienen die elektrischen Verbindungsmittel einer seitlichen Lasche zur Übertragung physiologischer Signale und die Verbindungsmittel der anderen seitlichen Lasche zur Stomversorgung.

Diese Maßnahme hat den Vorteil, daß trotz Verwendung gleicher Kontaktelemente eine Verwechslung der jeweiligen Anschlüsse nicht vorkommen kann.

Schließlich bestehen bei einer weiteren Ausgestaltung der Erfindung die Geräteteile aus einem individuellen Oberteil sowie einem jeweils gleichen Mittelteil und Unterteil, wobei die Laschen sowie die Kehlen an den Mittelteilen angeordnet sind.

Diese Maßnahme hat den Vorteil, daß Herstellung und Wartung der Geräte erheblich erleichtert werden, weil mit Ausnahme des Oberteils, das für die jeweilige Gerätefunktion unterschiedlich sein muß, im übrigen identische Teile verwendet werden. Weiterhin haben diese Maßnahmen den Vorteil, daß unterschiedliche Geräteteile in gleiche Halterungen oder Fächer von Notarztwagen, Hubschraubern u. dgl. eingesetzt werden können, weil die Breite und Tiefe der Teile übereinstimmen.

Weitere Vorteile ergeben sich aus der Beschreibung und der beigefügten Zeichnung.

Ausführungsbeispiele der Erfindung sind in der Zeichnung dargestellt und werden in der nachfolgenden Beschreibung näher erläutert. Es zeigen:

Fig. 1 eine perspektivische Ansicht von schräg oben eines Ausführungsbeispiels eines erfindungsgemäßen Gerätes;

Fig. 2 und 3 Schnittdarstellungen in zwei unterschiedlichen Ebenen einer Ausführungsform von elektrischen und mechanischen Verbindungsmitteln, wie sie beim erfindungsgemäßen Gerät verwendet werden können;

Fig. 4 einen Stromlaufplan zur Erläuterung der Wirkungsweise der elektrischen Verbindungsmittel;

Fig. 5 eine Darstellung wie Fig. 3, jedoch für ein weiteres Ausführungsbeispiel der Erfindung;

Fig. 6 und 7 Ansichten einer Lasche bzw. einer Haube in Richtung der Pfeile VI und VII von Fig. 5.

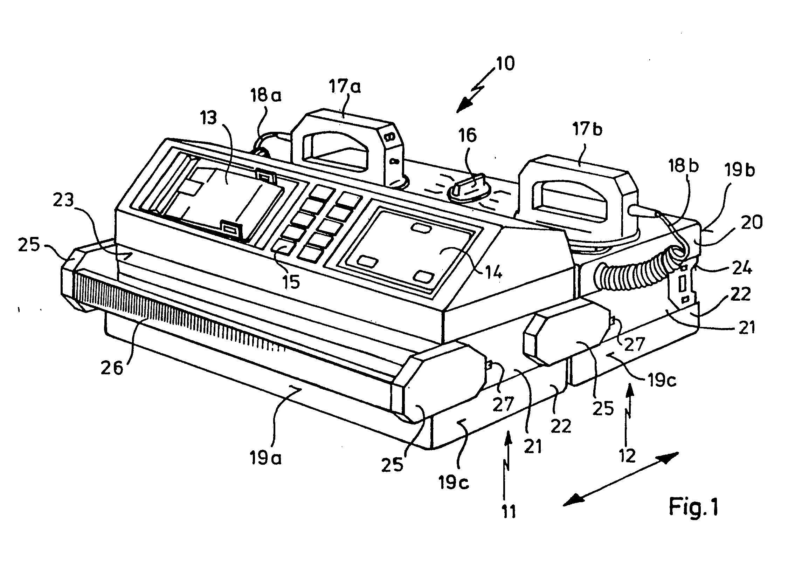

In Fig. 1 bezeichnet 10 insgesamt ein Ausführungsbeispiel eines erf, indungsgemäßen tragbaren elektromedizinischen Gerätes. Das Ausführungsbeispiel gemäß Fig. 1 ist aus zwei Geräteteilen zusammengesetzt, nämlich einer Monitor-Schreibereinheit 11 und einem Defibrillator 12. Die Einheit 11 weist in der linken oberen Ecke einen Schreiber 13 und in der rechten oberen Ecke eine Monitor-Anzeigeeinheit 14 auf. Zwischen diesen letztgenannten Elementen befinden sich Funktionstasten 15. Mit den Funktionstasten 15 können der Schreiber 13 und die Monitor-Anzeigeeinheit 14 gesteuert werden, beispielsweise zum Aufzeichnen eines EKG-Signales, zum Darstellen dieses EKG-Signales auf einer Bildröhre des Monitors oder zum Anzeigen von Zahlenwerten der Herzfrequenz oder zum Einstellen eines unteren und eines oberen Grenzwertes der Herzfrequenz, um Tachykardien und Bradykardien erkennen und durch ein Signal anzeigen zu können.

Der Defibrillator 12 verfügt über einen Einstellschalter 16, mit dem die vom Defibrillator abgegebene Energie vorgewählt werden kann. Auf die Oberseite des Defibrillators 12 sind zwei Elektroden 17a, 17b aufgesetzt, die über flexible Spiralkabel 18a, 18b mit dem Defibrillator 12 verbunden sind.

Es versteht sich, daß die in Fig. 1 gezeigte Kombination eines Gerätes 10 nur beispielhaft zu verstehen sind. Im Rahmen der vorliegenden Erfindung können selbstverständlich auch andere Kombinationen elektromedizinischer Geräte, einschließlich Schrittmacher, Atemfrequenzmonitoren, Temperaturmonitoren u. dgl. miteinander kombiniert werden.

Man kann weiter aus Fig. 1 erkennen, daß die beiden Geräteteile jeweils aus einem Oberteil 20, einem Mittelteil 21 und einem Unterteil 22 bestehen. Das Oberteil 20 ist für jedes Geräteteil individuell ausgebildet, bei der Monitor-Schreibereinheit 11 läuft das Oberteil 20 in die bereits erwähnten Komponenten 13 bis 15 aus, während das Oberteil 20 des Defibrillators 12 im wesentlichen zur Aufnahme der Elektroden 17a, 17b bestimmt und ausgebildet ist.

Die Mittelteile 21 und die Unterteile 22 hingegen sind bei beiden Geräteteile vollkommen identisch ausgebildet, dies kann auch für den Innenaufbau dieser Teile gelten, beispielsweise dadurch, daß die Unterteile 22 mit baugleichen Netzgeräten versehen sind und die Mittelteile 21 zumindest mit baugleichen Halterungen für verschiedenartige Platinen von elektronischen Schalteinheiten.

Die in Fig. 1 dargestellten Geräteteile verfügen jeweils über eine Vorderwand 19a, eine Rückwand 19b sowie Seitenwände 19c. Im Bereich der Vorderwand 19a und der Rückwand 19b ist jedes Mittelteil 21 mit einer vorderen Kehle 23 bzw. einer hinteren Kehle 24 versehen. Die Kehlen 23, 24 haben näherungsweise einen trapezförmigen Querschnitt.

Auf die Seitenwände 19c der Mittelteile 21 sind ferner im an die Vorderwände 19a angrenzenden Bereich seitliche Laschen 25 aufgesetzt, die etwa zur Hälfte über die Vorderwand 19a vorstehen. Im dargestellten Ausführungsbeispiel ist die lichte Weite zwischen den freien Enden der Laschen 25 daher so groß wie die Breite der Vorderwand 19a. Es ist jedoch selbstverständlich auch möglich, die Laschen 25 außen mit den Seitenwänden 19c bündig zu machen und die Seitenwände 19c in dem an die Rückwand 19b angrenzenden Bereich seitlich mit entsprechenden Ausnehmungen zu versehen, so daß die lichte Weite zwischen den freien Enden der Laschen 25 auch kleiner sein kann als die Breite der Vorderwand 19a, was damit insgesamt zu einer von Vorsprüngen freien Seitenwand 19c führen würde.

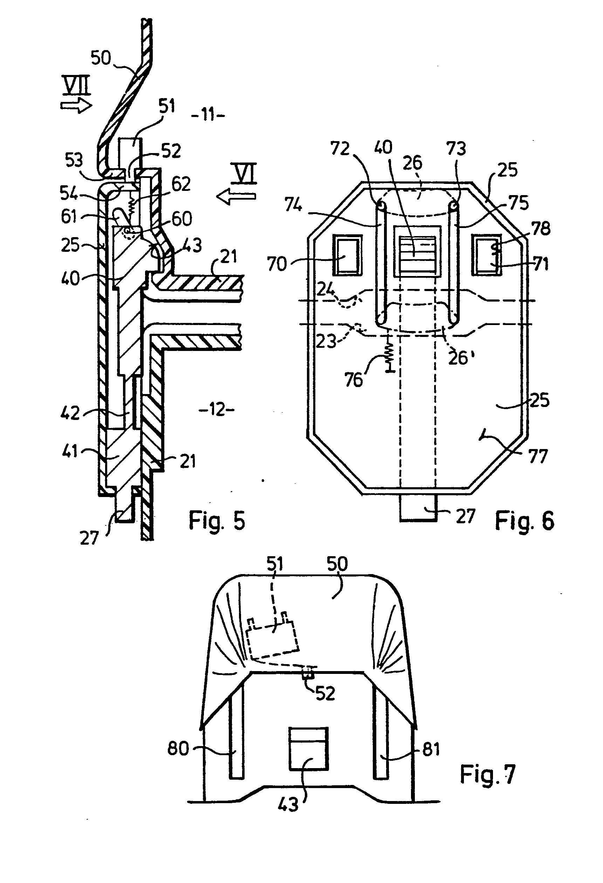

Beim Ausführungsbeispiel gemäß Fig. 1 ist zwischen den freien Enden der Laschen 25 ein durchgehender Griff 26 angeordnet, mit dem das Gerät 10 insgesamt ergriffen und getragen werden kann. Außerdem sind am entgegengesetzten Ende der Laschen 25 Knöpfe 27 zu erkennen, die zum Lösen der Verbindung zwischen den Geräteteilen dienen, wie weiter unten zu Fig. 5 noch im einzelnen beschrieben werden wird.

Wie weiter unten ebenfalls noch im einzelnen erläutert werden wird, sind die Laschen 25 an ihrer freiliegenden Innenseite mit elektrischen und mechanischen Verbindungsmitteln versehen, die mit entsprechenden Verbindungsmitteln zusammenarbeiten, die in dem an die Rückwand 19b angrenzenden Bereich auf der Seitenwand 19c der Mittelteile 21 angeordnet sind. Zum Verbinden zweier Geräteteile, beispielsweise der Monitor-Schreibereinheit 11 und des Defibrillators 12 in Fig. 1, ordnet man diese Geräteteile daher zunächst hintereinander an und schiebt sie in die in Fig. 1 dargestellte Stellung entlang des rechts unten in Fig. 1 dargestellten Pfeiles zusammen. Wie weiter unten noch gezeigt werden wird, verschwindet dabei der Griff 26 des Defibrillators 12 in dem Zwischenraum, der von den Kehlen 23, 24 gebildet wird.

Will man hingegen die Geräteteile wieder separat verwenden, genügt es, ein oder zwei Knöpfe 27 zu drücken und die Geräteteile in Pfeilrichtung auseinanderzuziehen.

Während im verbundenen Zustand durch die elektrischen Verbindungsmittel alle notwendigen Signalverbindungen hergestellt und die Stromversorgungen so miteinander verkoppelt wurden, daß das Gesamtgerät notfalls auch aus der Stromversorgung eines einzelnen Geräteteils betrieben werden kann, sind diese Verbindungen bei getrennten Geräteteilen naturgemäß wieder unterbrochen, und jedes Geräteteil ist für sich voll funktionsfähig.

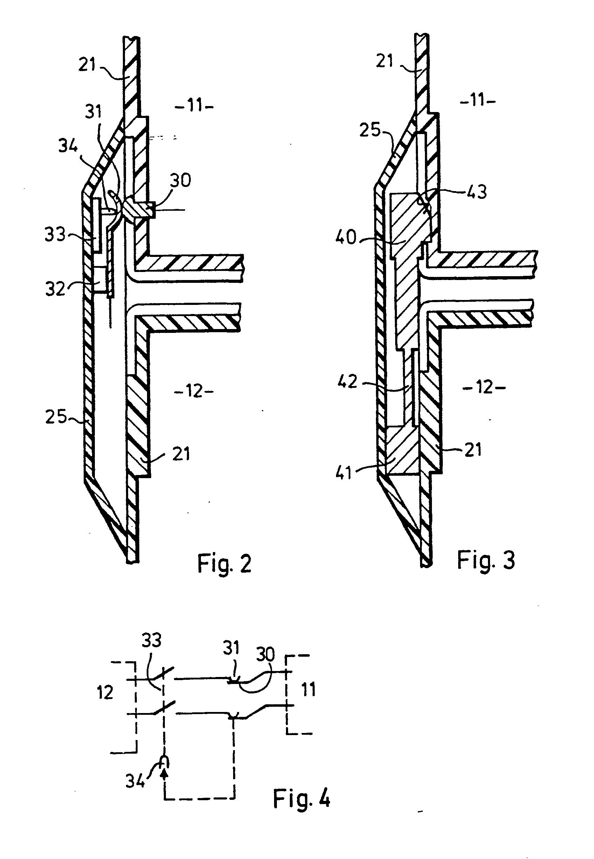

In den Fig. 2 und 3 ist eine Ausführungsform von elektrischen und mechanischen Verbindungsmitteln dargestellt, wie sie beim Gerät gemäß Fig. 1 verwendet werden kann. Die Schnittdarstellung gemäß Fig. 2 zeigt eine Lasche 25 in einer Schnittebene etwas unterhalb bzw. oberhalb der Längsmittelebene der Lasche 25, während die Darstellung von Fig. 2 etwa durch die Längsmittelebene der Lasche 25 geht.

In Fig. 2 erkennt man, daß in das Mittelteil 21 der Monitor-Schreibeinheit 11 eine Kontaktniet 30 eingelassen ist, die bei Verbindung mit einem Defibrillator 12 mit einer elastischen Kontaktfeder 31 zusammenwirkt, die ihrerseits auf einem Sockel 32 innen an der Lasche 25 befestigt ist. Beim Zusammenschieben der Geräteteile läuft die Kontaktfeder 31 auf den Kopf der Kontaktniet 30 auf und wird etwas nach links in Fig. 2 ausgelenkt. Dies macht man sich für eine Erkennung der vollzogenen Verbindung zunutze, indem man einen Mikroschalter 33 ebenfalls auf der Innenseite der Lasche 25 anbringt, dessen Schaltfühler 34 von innen an der Kontaktfeder 31 anliegt. Wird nun die Kontaktfeder 31 — wie geschildert — nach links in Fig. 2 ausgelenkt, wird der Mikroschalter 33 betätigt.

Es versteht sich, daß die in Fig. 2 dargestellten elektrischen Verbindungsmittel auch in größerer Anzahl vorhanden sein können, je nachdem, wieviel Signal- und Versorgungsverbindungen erforderlich sind.

In Fig. 3 erkennt man die mechanischen Verbindungsmittel dieses Ausführungsbeispiels in Gestalt einer Rastnase 40, die auf einem Sockel 41 in der Lasche 25 befestigt ist. Zwischen Sockel 41 und Rastnase 40 liegt ein verjüngter Abschnitt 42, so daß die Rastnase 40 in der Zeichenebene von Fig. 3 elastisch nach links ausweichen kann. Wird nun der Defibrillator 12 von unten in Fig. 3 mit der Monitor-Schreibereinheit 11 verbunden, weicht die Rastnase 40 zunächst nach links aus und federt dann in eine Aussparung 43 im Mittelteil 21 der Monitor-Schreibereinheit 11. Die Mittel zum Lösen der Rastverbindung sind in Fig. 3 nicht dargestellt und werden weiter unten in Fig. 5 anhand eines Beispiels noch erläutert.

Fig. 4 zeigt in Gestalt eines vereinfachten Stromlaufplanes die elektrische Verbindung zwischen Defibrillator 12 und Monitor-Schreibereinheit 11. Man erkennt, daß die Kontakte des Mikroschalters 33 in Reihe mit den elektrischen Verbindungsmitteln, nämlich dem aus der Kontaktniet 30 und der Kontaktfeder 31 gebildeten Kontakt liegen. Die gestrichelte Linie deutet dabei an, daß über die Kontaktfeder 31 in der beschriebenen Weise der Schaltfühler 34 und damit der Mikroschalter 33 betätigt wird. Bei geöffneten Kontakten des Mikroschalters 33 sind die beispielsweise als Signaleingänge dienenden Kontaktfedern 31 elektrisch abgetrennt, so daß keine Störstrahlungen in den Defibrillator 12 gelangen und dort beispielsweise zu einer unsynchronisierten Auslösung des Elektroschocks führen können. Es versteht sich, daß — obwohl in Fig. 4 nicht dargestellt — entsprechende Mikroschalter auch auf seiten der Monitor-Schreibereinheit 11 vorgesehen sein können.

Fig. 5 zeigt eine Variante der Darstellung von Fig. 3, bei der das Mittelteil 21 der Monitor-Schreibereinheit 11 mit einer Haube 50 versehen ist, die im verbundenen Zustand der Geräteteile mit einer Stirnwand 53 an eine gegenüberliegende Stirnwand 54 der Lasche 25 angrenzt. In der Stirnwand 53 der Haube 50 befindet sich eine Öffnung für einen Schaltfühler 52 eines Mikroschalters 51. Beim Zusammenschieben und damit Verbinden der beiden Geräteteile drückt demzufolge die Stirnwand 54 der Lasche 25 auf den Schaltfühler 52 und betätigt damit den Mikroschalter 51, der so geschaltet sein kann, wie dies in Fig. 4 für den Mikroschalter 33 dargestellt wurde.

Man erkennt in Fig. 5 weiter, daß die elastische Rastnase 40 seitlich mit einem Stift 60 versehen ist, der in einer zur Verbindungsrichtung schrägen Nutführung 61 läuft. Außerdem ist zwischen Rastnase 40 und Stirnwand 54 eine Druckfeder 62 angeordnet. Der die Rastnase 40 tragende Sockel 41 läuft an dem von der Rastnase 40 abgewandten Ende in den Knopf 27 aus.

Will man die Monitor-Schreibereinheit 11 wieder vom Defibrillator 12 lösen, genügt es, den Knopf 27 in Fig. 5 gegen die Kraft der Druckfeder 62 nach oben zu drücken. Durch die schräge Nutführung 61 und den Stift 60 gleitet nun die Rastnase 40 nach links oben, so daß sie außer Eingriff mit der Aussparung 43 gerät und damit der Defibrillator 12 von der Monitor-Schreibereinheit 11 abgezogen werden kann, läßt man den Knopf 27 wieder los, sorgt die Druckfeder 62 dafür, daß die Rastnase 40 wieder in ihre Ausgangsstellung nach rechts gelangt und beim nächsten Verbinden mit einem anderen Geräteteil wieder für die erforderliche Rastverbindung sorgt.

Fig. 6 zeigt eine Ansicht der Lasche 25 in Richtung des Pfeiles VI von Fig. 5. Man erkennt zunächst zwei seitlich von der Mitte angeordnete Kontakte 70, 71, die beispielsweise so wie die Kontaktfeder 31 gemäß Fig. 2 ausgebildet sein können. Man erkennt ferner am oberen Rand der Lasche 25 gestrichelt eingezeichnet den Querschnitt des Griffes 26 in der Stellung, die der Griff 26 der Monitor-Schreibereinheit 11 von Fig. 1 einnimmt. Der Griff 26 ist seitlich mit Stiften 72, 73 versehen, die in einer Nutführung 74 bzw. 75 der Lasche 25 laufen, und zwar in einer Richtung, die der Verbindungsrichtung zweier Geräteteile beim Zusammenfügen entspricht. Der Griff 26 bewegt sich in diesem Fall in Fig. 6 nach unten, gleitet über die elastisch nach hinten in Fig. 6 zurückfedernde Rastnase 40 und gelangt schließlich in eine untere Stellung, die in Fig. 6 mit 26' bezeichnet ist und die innerhalb des von den Kehlen 23, 24 begrenzten Hohlraums liegt. Der Griff 26 bewegt sich dabei gegen die Kraft einer in Fig. 6 nur schematisch dargestellten Druckfeder 76, so daß beim Lösen der Geräteteile voneinander der Griff 26 von selbst wieder in die in Fig. 6 oben eingezeichnete Grundstellung gelangt.

Die Lasche 25 kann auf der in Fig. 1 nach innen weisenden Seite mit einem Deckel 77 versehen sein, in den die Führungsnuten 74, 75 eingelassen sind. Außerdem sind im Deckel 77 Fenster 78 für die Kontakte 70, 71 und die Rastnase 40 vorgesehen, so daß alle in der Lasche 25 befindlichen Elemente optimal geschützt sind.

Fig. 7 zeigt schließlich noch eine Ansicht der Haube 50 in Richtung des Pfeiles VII von Fig. 5.

Man erkennt zunächst in dem von der Haube 50 verdeckten Teil den Mikroschalter 51 mit dem Schaltfühler 52. Unterhalb der Haube 50 erkennt man die Aussparung 43 für die Rastnase 40 und seitlich davon zwei Kontaktbahnen 80, 81, die mit den Kontakten 70, 71 von Fig. 6 zusammenarbeiten. Es versteht sich jedoch, daß statt der Kontaktbahnen 80, 81 auch Kontaktnieten nach Art der Kontaktniet 30 von Fig. 2 verwendet werden können.

Statt der Federkontakte 70, 71/80, 81 können auch Steckerkontakte verwendet werden. Hierzu kann die Lasche 25 in Fig. 5 am oberen Ende so ausgebildet sein, daß sie im zusammengefügten Zustand an oder unter oder über die laschenartig ausgebildete Haube 50 bzw. deren Stirnwand 53 reicht, wobei an der Stirnwand 53 bzw. der dann angrenzenden Stirnwand 54 der Lasche 25 Steckerkontakte angebracht sind, so wie dies entsprechend für den Mikroschalter 51 in Fig. 5 gezeigt ist.

## Patentansprüche

1. Tragbares elektromedizinisches Gerät (10), bestehend aus mehreren Geräteteilen, beispielsweise einer Monitor-Schreibereinheit (11) und einem Defibrillator (12), wobei die Geräteteile mindestens näherungsweise quaderförmig mit einer Vorderwand (19a), einer Rückwand (19b) und zwei Seitenwänden (19c) ausgebildet und im Bereich der Wände (19a bis 19c) mittels Steckverbindungen mechanisch und elektrisch verbindbar sind, dadurch gekennzeichnet, daß die Seitenwände (19c) im an die Vorderwand (19a) angrenzenden Bereich mit seitlichen, Teile von Steckverbindungen bildenden Laschen (25) versehen sind, die über die Vorderwand (19a) vorstehen und dort an ihren freien Enden über einen durchgehenden Griff (26) verbunden sind, daß die Vorderwand (19a) und/oder die Rückwand (19b) mit einer parallel zum Griff (26) verlaufenden Kehle (23, 24) versehen ist und daß der Griff (26) in den Laschen (25) verschiebbar angeordnet ist, derart, daß beim Verbinden zweier Geräteteile die freien Enden der Laschen (25) eines Geräteteiles die Seitenwände (19c) des anderen Geräteteiles umfassen und der Griff (26) in den Bereich der Kehle (23, 24) gelangt sodaß die Geräteteile fest miteinander verbunden sind.

2. Gerät nach Anspruch 1, dadurch gekennzeichnet, daß der Griff (26) beim Verbinden zweier Geräteteile gegen die Kraft einer Feder (76) geführt wird.

3. Gerät nach Anspruch 1 oder 2, dadurch gekennzeichnet, daß der Griff (26) mit an seinen Enden angeordneten Stiften (72, 73) in Führungsnuten (74, 75) der Laschen (25) läuft.

4. Gerät nach einem der Ansprüche 1 bis 3, dadurch gekennzeichnet, daß die Laschen (25) mit mechanischen und/oder elektrischen Verbindungsmitteln versehen sind, die mit zugeordneten mechanischen und/oder elektrischen Verbindungsmitteln im an die Rückwand (19b) angrenzenden Bereich der Seitenwände (19c) eines anderen Geräteteiles zusammenarbeiten.

5. Gerät nach Anspruch 4, dadurch gekennzeichnet, daß die mechanischen Verbindungsmittel als mindestens eine elastische Rastnase (40) in einem Geräteteil ausgebildet sind, die mit einer Aussparung (43) in einem anderen Geräteteil zusammenarbeitet.

6. Gerät nach Anspruch 5, dadurch gekennzeichnet, daß die Rastnasen (40) in den Laschen (25) angeordnet sind, wobei die Rastnasen (40) in einer Führung (61) schräg zur Verbindungsrichtung der Geräteteile mittels eines Knopfes (27) gegen die Kraft einer Feder (62) außer Eingriff mit der Aussparung (43) bewegbar sind.

7. Gerät nach einem der Ansprüche 4 bis 6, dadurch gekennzeichnet, daß die elektrischen Verbindungsmittel als Kontaktfedern (31) bzw. Kontaktstecker in einem Geräteteil ausgebildet sind, die mit Kontaktnieten (30) oder Kontaktbahnen (80, 81) bzw. Kontaktbuchsen in einem anderen Geräteteil zusammenarbeiten.

8. Gerät nach einem der Ansprüche 4 bis 7, dadurch gekennzeichnet, daß im Bereich der Lasche (25) ein beim Verbinden zweier Geräteteile betätigter Schalter angeordnet ist, dessen Schaltkontakte in Reihe mit den elektrischen Verbindungsmitteln liegen.

9. Gerät nach Anspruch 7 und 8, dadurch gekennzeichnet, daß der Schalter als Mikroschalter (33) ausgebildet ist, dessen Schaltfühler (34) mit der Kontaktfeder (31) ausgelenkt wird.

10. Gerät nach Anspruch 8, dadurch gekennzeichnet, daß der Schalter als Mikroschalter (51) ausgebildet ist, dessen Schaltfühler (52) beim Zusammenlaufen der Lasche (25) eines Geräteteiles mit einer auf einem anderen Geräteteil angeordneten Haube (50), die bei verbundenen Geräteteilen an die Lasche (25) angrenzt, ausgelenkt wird.

11. Gerät nach einem der Anprüche 4 bis 10, dadurch gekennzeichnet, daß die elektrischen Verbindungsmittel einer seitlichen Lasche (25) zur Übertragung physiologischer Signale und die Verbindungsmittel der anderen seitlichen Lasche (25) zur Stromversorgung dienen.

12. Gerät nach einem der Ansprüche 1 bis 11, dadurch gekennzeichnet, daß die Geräteteile aus einem individuellen Oberteil (20) sowie einem jeweils gleichen Mittelteil (21) und Unterteil (22) bestehen und daß die Laschen (25) sowie die Kehlen (23, 24) an den Mittelteilen (21) angeordnet sind.

## Revendications

1. Appareil médical électroportatif (10) se composant de plusieurs sous-ensembles d'appareil, par exemple d'une unité moniteur-imprimante (11) et d'un défibrillateur (12) dans lequel les sous-ensembles d'appareils sont de forme approximativement parallélépipédique et comportent au moins une paroi frontale (19a), une paroi arrière (19b) et deux parois latérales (19c) et sont accouplables au moyen de liaisons (connexions) mécaniques et électriques à emmanchement, caractérisé

en ce que les parois latérales (19c) sont pourvues, dans la zone adjacente de la paroi frontale (19a) d'éclisses latérales (25) faisant partie de la liaison à emmanchement, qui font saillie au-delà de la paroi frontale et y sont reliées par leurs extrémités libres au moyen d'une poignée traversante (26).

en ce que la paroi frontale (19a) et/ou la paroi arrière (19b) présente une gorge (23, 24) parallèle à la poignée,

et en ce que la poignée (26) est montée coulissante dans les éclisses (25) de façon que, lors de l'accouplement des deux sous-ensembles d'appareils, les éclisses (25) aux extrémités libres de l'un des sous-ensembles d'appareils viennent enserrer les parois (19c) de l'autre sous-ensemble d'appareil et la poignée (26) atteint la zone des gorges (23, 24) de façon à relier solidairement un appareil à l'autre.

2. Appareil selon la revendication 1, caractérisé en ce que, lors de l'accouplement, la poignée (26) est déplacée contre la force de rappel d'un ressort (76).

3. Appareil selon les revendications 1 ou 2, caractérisé en ce que la poignée (26) comporte deux nervures de renforcement (72, 73) à ses extrémités qui se déplacent dans des rainures de guidage (74, 75) des éclisses.

4. Appareil selon l'une des revendications de 1 à 3, caractérisé en ce que les éclisses (25) sont pourvues de moyens de connexions mécaniques et/ou électriques qui fonctionnent en association avec les moyens de connexions mécaniques et/ou électriques prévus sur la paroi arrière (19b) dans la zone adjacente des parois latérales (19c).

5. Appareil selon la revendication 4, caractérisé en ce que les moyens de connexion mécanique consistent en au moins une saillie butée (40) conformée dans l'un des sous-ensembles d'appareil qui fonctionnent avec une rainure (43) d'un autre sous-ensemble d'appareil.

6. Appareil selon la revendication 5, caractérisé en ce que les saillies-butées (40) sont prévues dans les éclisses (25) de façon que les saillies-butées (40) se déplacent suivant un guidage oblique (61) par rapport à la direction d'accouplement des sous-ensembles d'appareils par l'intermédiaire d'un ergot (27) contre la force de rappel d'un ressort (62) mobile extérieurement et enserrant la rainure (43).

7. Appareil selon l'une des revendications de 4 à 6, caractérisé en ce que les moyens d'accouplement électrique sont des ressorts de contact (31) notamment une fiche-contact conformée dans l'un des sous-ensembles d'appareil qui fonctionne en association avec l'autre appareil par des contacts-rivets (30) ou des contacts à piste (80, 81) notamment des contacts à bornes.

8. Appareil selon l'une des revendications de 4 à 7, caractérisé en ce que, dans la région de l'éclisse (25) est prévu un interrupteur qui est actionné par l'accouplement des sous-ensembles d'appareils, interrupteur dont les contacts sont en alignement avec les moyens d'accouplement électrique.

9. Appareil selon les revendications 7 et 8, caractérisé en ce que l'interrupteur est un micro-interrupteur (33) dont le doigt de commande (34) est relié mécaniquement au ressort de contact 31.

10. Appareil selon la revendication 8, caractérisé en ce que le doigt de commande (52) de l'interrupteur réalisé sous la forme d'un micro-interrupteur (51) est déconnecté lors du mouvement d'accouplement de l'éclisse (25) d'un sous-ensemble d'appareil par le capot (50) prévu sur l'autre sous-ensemble d'appareil.

11. Appareil selon l'une des revendications de 4 à 10, caractérisé en ce que les moyens d'accouplement électrique d'une des éclisses latérales (25) servent au transfert du signal psychologique et les moyens d'accouplement de l'autre éclisse latérale (25) servent à l'alimentation électrique.

12. Appareil selon l'une des revendications 1 à 11, caractérisé en ce que les sous-ensembles d'appareil comprennent une partie supérieure (20) individuelle, ainsi qu'une partie médiane (21) et inférieure (22) identiques, et en ce que les éclisses (25) ainsi que les gorges (23, 24) sont prévues dans la partie médiane.

## Claims

1. A portable electromedical apparatus (10) comprising a plurality of apparatus portions, for example a monitor recorder unit (11) and a defibrillator (12), wherein the apparatus portions are at least approximately cuboids with a front wall (19a), a rear wall (19b) and two side walls (19c) and can be mechanically and electrically connected in the region of the walls (19a to 19c) by means of plug connections characterised in that, in the region adjoining the front wall (19a), the side walls (19c) are provided with side plates (25) which form parts of plug connections and which project beyond the front wall (19a) and are there connected at their front ends by way of a continuous handle (26), that the front wall (19a) and/or the rear wall (19b) is provided with a channel (23, 24) which extends parallel to the handle (26) and that the handle (26) is arranged displaceably in the side plates (25) in such a way that when two apparatus portions are connected the free ends of the side plates (25) of one apparatus portion embrace the side walls (19c) of the other apparatus portion and the handle (26) passes into the region of the channel (23, 24) so that the apparatus portions are fixedly connected together.

2. Apparatus according to claim 1 characterised in that, when two apparatus portions are connected, the handle (26) is moved against the force of a spring (76).

3. Apparatus according to claim 1 or claim 2 characterised in that the handle (26) runs in guide grooves (74, 75) of the side plates (25), with pins (72, 73) which are arranged at the ends of the handle.

4. Apparatus according to one of claims 1 to 3 characterised in that the side plates (25) are provided with mechanical and/or electrical connecting means which co-operate with associated mechanical and/or electrical connecting means in the region, which adjoins the rear wall (19b), of the side walls (19c) of another apparatus portion.

5. Apparatus according to claim 4 characterised in that the mechanical connecting means are in the form of at least one resilient retaining projection (40) in one apparatus portion, which retaining projection co-operates with an opening (43) in another apparatus portion.

6. Apparatus according to claim 5 characterised in that the retaining projections (40) are arranged in the side plates (25), wherein the retaining projections (40) are movable in a guide (61) inclinedly with respect to the direction of connection of the apparatus portions by means of a knob (27) against the force of a spring (62) to come out of engagement with the opening (43).

7. Apparatus according to one of claims 4 to 6 characterised in that the electrical connecting means are in the form of contact springs (31) or contact plugs in an apparatus portion, which co-operate with contact rivets (30) or contact paths (80, 81) or contact sockets in another apparatus portion.

8. Apparatus according to one of claims 4 to 7 characterised in that disposed in the region of the side plate (25) is a switch which is actuated when two apparatus portions are connected together and the switching contacts of which are in series with the electrical connecting means.

9. Apparatus according to claim 7 and claim 8 characterised in that the switch is in the form of a microswitch (33) having a switching sensor (34) which is deflected with the contact spring (31).

10. Apparatus according to claim 8 characterised in that the switch is in the form of a microswitch (51) having a switching sensor (52) which is deflected when the side plate (25) of an apparatus portion meets a cover (50) which is arranged on another apparatus portion and which adjoins the side plate (25) when the apparatus portions are connected together.

11. Apparatus according to one of claims 4 to 10 characterised in that the electrical connecting means of a side plate (25) serve for the transmission of physiological signals and the connecting means of the other side plate (25) serve for the power supply.

12. Apparatus according to one of claims 1 to 11 characterised in that the apparatus portions comprise an individual upper portion (20) and respectively identical middle portion (21) and lower portion (22) and that the side plates (25) and the channels (23, 24) are arranged on the middle portions (21).

Fig.1

EP 0 167 122 B1

Fig. 2

Fig. 3

Fig. 4

Fig. 5

Fig. 6

Fig. 7